(19) Europäisches Patentamt
European Patent Office
Office européen des brevets

(11) Veröffentlichungsnummer: **0 162 395**
**B1**

(12) **EUROPÄISCHE PATENTSCHRIFT**

(45) Veröffentlichungstag der Patentschrift: **18.04.90**

(51) Int. Cl.$^5$: **C 07 D 501/46**

(21) Anmeldenummer: **85105839.6**

(22) Anmeldetag: **13.05.85**

(54) **Verfahren zur Herstellung von Cephalosporinen.**

(30) Priorität: **22.05.84 DE 3419015**

(43) Veröffentlichungstag der Anmeldung:
**27.11.85 Patentblatt 85/48**

(45) Bekanntmachung des Hinweises auf die Patenterteilung:
**18.04.90 Patentblatt 90/16**

(84) Benannte Vertragsstaaten:
**AT BE CH DE FR GB IT LI SE**

(56) Entgegenhaltungen:
**EP-A-0 023 453**

(73) Patentinhaber: **BAYER AG**
**D-5090 Leverkusen 1 Bayerwerk (DE)**

(72) Erfinder: **Angerbauer, Rolf, Dr.**
**Sterntalerweg 29**
**D-5600 Wuppertal 1 (DE)**
Erfinder: **Kinast, Günther, Dr.**
**Am Eckbusch 15a**
**D-5600 Wuppertal 1 (DE)**

**Die Akte enthält technische Angaben, die nach dem Eingang der Anmeldung eingereicht wurden und die nicht in dieser Patentschrift enthalten sind.**

**EP 0 162 395 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft ein Verfahren zur Herstellung bekannter Cephalosporine der allgemeinen Formel I,

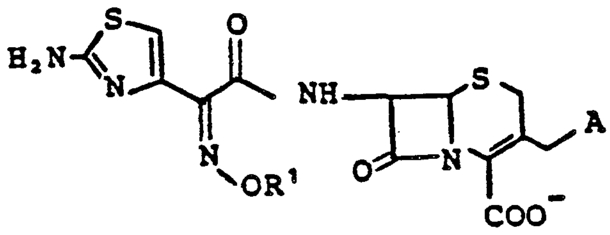

(I)

die u.a. in

EP 64 740
EP 74 645
DE—OS 33 11 300
US—PS 4 406 899

genannt sind und in denen

$R^1$ einen $C_1$—$C_6$-Alkyl-Rest darstellt, der geradkettig, verzweigt, cyclisch und auch ungesättigt sein kann und

A einen Rest der Formeln

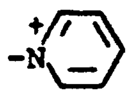
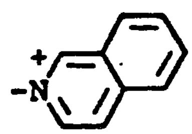
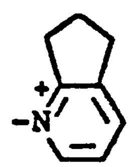
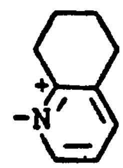

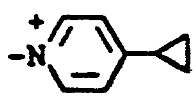
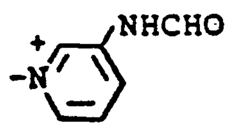
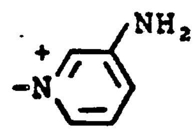

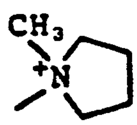
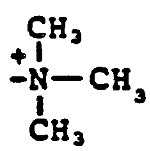

darstellt.

Das Verfahren ist dadurch gekennzeichnet, daß man eine Säure der allgemeinen Formel II, mit Methansulfonsäurechlorid in das gewünschte Anhydrid III überführt und dieses mit einem 7-Aminocephalosporanat der allgemeinen Formel IV umsetzt, wobei A die oben genannte Bedeutung hat.

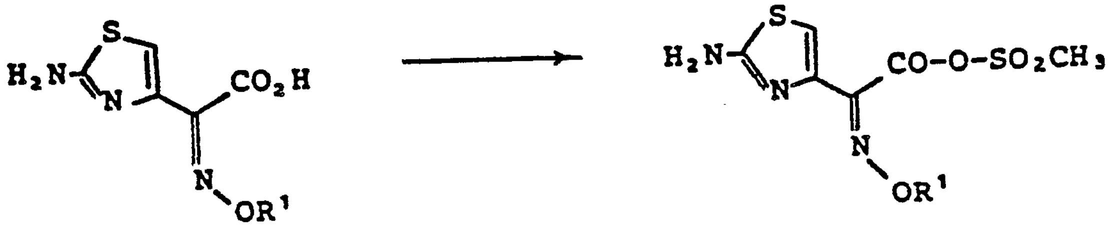

(II) (III)

(III) + 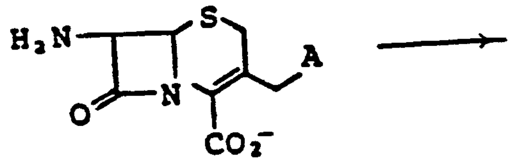 (I)

(IV).

Zum Stand der Technik gehört die EP—A—0 023 453, die ein Verfahren zur Herstellung von 7-[2-Aryl-2-hydroximino-acetamido]-cephalosporansäurederivaten aus den entsprechenden 7-Aminoverbindungen mittels gemischter Carbonsäure-sulfonsäureanhydride beschreibt.

Die erfindungsgemäßen gemischten Anhydride werden hergestellt, indem man die Carbonsäure der Formel II und 1-1,1 Äquivalent eines Amins in einem Lösungsmittel löst und mit 1-1,2 Äquivalenten Methansulfonsäurechlorid reagieren läßt.

Als Lösungsmittel eignen sich alle Solventien, die unter den Reaktionsbedingungen stabil sind, wie z.B. Diethylether, Tetrahydrofuran, Acetonitril, Aceton, Methylenchlorid, Chloroform, Dimethylformamid oder Gemische derselben.

Als Amine eignen sich tertiäre Amine wie z.B. Triethylamin, Tripropylamin, Tributylamin, Ethyldiisopropylamin, sterisch gehinderte sekundäre Amine wie z.B. Diisopropylamin oder Gemische dieser Amine.

Die Reaktionstemperatur kann zwischen −80°C und Raumtemperatur liegen, wobei tiefe Temperaturen besonders bei Verwendung von Dimethylformamid von Vorteil sind.

Für die nachfolgende Umsetzung des gemischten Anhydrids III mit 7-Aminocephalosporanaten IV ist es aus Löslichkeitsgründen vorteilhaft, die Anhydride III in Dimethylformamid bei −40 bis −60°C und einer Reaktionsdauer von 0,2 bis 24 h, bevorzugt 0,5—2 h, herzustellen und die erhaltene Lösung des gemischten Anhydrids III direkt mit einer Lösung von 0,5—2,0 Äquivalenten der 7-Aminocephalosporanate IV in einem geeigneten Lösungsmittel und einer geeigneten Base zu den gewünschten Produkten I umzusetzen.

Für die genannte Umsetzung von III und IV nach I werden die 7-Aminocephalosporanate IV aus Löslichkeitsgründen in einem polaren Lösungsmittel wie Dimethylformamid, Dimethylsulfoxid, Diglyme oder Wasser, bevorzugt in Wasser gelöst. Die Lösungen von III und von IV und die Base werden bei einer geeigneten Temperatur zusammengegeben und bis zum Ende der Reaktion kräftig gerührt.

Als Base können die oben gennanten tertiären und sterisch gehinderten sekundären Amine dienen, die mit 1-2,5 Äquivalenten, bezogen auf das Anhydrid III, eingesetzt werden. Bevorzugt wird Triethylamin verwendet. Als Base können aber auch anorganische Basen wie NaOH, KOH, $NaHCO_3$ oder $K_2CO_3$ und deren wäßrige Lösungen verwendet werden. Der pH sollte während der Reaktion über 7 liegen und kann je nach Stabilität des Endproduktes I kurzzeitig bis an 12 heranreichen. Höhere pH-Werte beschleunigen die Reaktion.

Die Reaktionstemperatur liegt zwischen −50°C und +50°C, wobei bei Umsetzungen bei pH-Werten über 9 Reaktionstemperaturen unter 0°C bevorzugt sind.

Die Reaktionsdauer hängt von der Reaktionstemperatur und dem pH-Wert ab und liegt zwischen wenigen Minuten und mehreren Stunden. Im allgemeinen ist die Umsetzung bei Temperaturen von −20 bis 0°C und einem pH von etwa 9 nach bis 20 Minuten beendet.

Die Aufarbeitung hängt von den Eigenschaften der Ausgangsverbindungen II und IV, des verwendeten Amins und der Basen und von den Eigenschaften der Produkte I ab. Im allgemeinen lassen sich die Produkte I nach Zugabe einer Säure und die entsprechenden Salze der Produkte I mit einem geeigneten Lösungsmittel wie z.B. Aceton, Ethanol oder Isopropanol aus der Reaktionslösung ausfällen oder auskristallisieren.

Besonders bevorzugt werden nach dem erfindungsgemäßen Verfahren Verbindungen der Formel I hergestellt, in denen R1 eine Methylgruppe darstellt.

Das beschriebene Verfahren zur Herstellung der Verbindungen der Formel I hat den Vorteil daß

1. die Aminogruppe in II nicht geschützt werden muß
2. die Z-konfigurierte $\overset{\|}{N}$ $OR^1$-Gruppierung nicht zur unerwünschten-E-Konfiguration isomerisiert
3. das zur Ativierung der Carbonsäuren II verwendete Reagenz Methansulfonsäurechlorid billig und gut handhabbar ist
4. die Reaktion von III mit IV durch Variation der Reaktionsbedingungen gut zu kontrollieren ist
5. und auch in wässrigen Systemen durchzuführen ist, was aus Löslichkeitsgründen der Verbn. IV besonders vorteilhaft ist
6. die Abtrennung der bei der Reaktion von III mit IV entstehenden Methansulfonsäure keine Probleme bereitet
7. die Reaktionsausbeuten an gewünschtem Produkt I sehr hoch liegen.

Beispiel 1

7β-[(Z)-2-(2-Aminothiazol-4-yl)-2-methoxyiminoacetamido]-3-pyridiniummethyl-3-cephem-4-carboxylat

37.66 g (0.187 Mol) 2-Aminothiazol-4-yl-methoxyiminoessigsäure und 34.2 ml (0.196 Mol) Ethyldiisopropylamin werden in 160 ml Dimethylformamid gelöst, auf −60°C abgekühlt und 15.1 ml (0.196 Mol) Methansulfonsäurechlorid hinzugegeben. Es wird 40 min. bei −60 bis −50°C gerührt und man gibt die Lösung in einem Guß zu einer auf 0°C vorgekühlten Lösung von 50 g (0.149 Mol) 7-Amino-2-pyridiniummethyl-2-cephem-3-carboxylat × 1 HCl × 1 $H_2O$ und 55 ml (0.40 Mol) Triethylamin in 54 ml Wasser und rührt 10 Minuten sehr kräftig ohne Kühlung. Dann wird der Ansatz in 7.5 l Aceton eingetragen, der Niederschlag abgesaugt, mit Aceton und Methylenchlorid gewaschen und getrocknet. Man erhält 66 g (96%) des gewünschten Produktes, das durch Lösen in 64 ml konzentrierter Salzsäure und Zugabe von 64 ml 2N Salzsäure und 250 ml Isopropanol in ein kristallines Dihydrochlorid überführt werden kann. Ausbeute an Dihydrochlorid: 60 g (76%).

$^1$H-NMR ($D_2O$) δ (ppm) = 9.04 (2H, d, J=7Hz, H-2,6-Py); 8.68 (1H, m, H-4-Py); 8.19 (2H, m, H-3,5-Py); 7.18 (1H, s, Thiazol); 5.93 (1H, d, J=5Hz, H-7-Lactam); 5.89 (1H, d, J=15Hz, $CH_2$-Py); 5.49 (1H, d, J=15Hz, $CH_2$-Py); 5.39 (1H, d, J=5Hz, H-6-Lactam); 4.10 (3H, s, $OCH_3$); 3.82 (1H, d, J=18Hz, S-$CH_2$); 3.43 (1H, d, J=18Hz, S-$CH_2$).

Beispiel 2

7β-[(Z)-2-(2-Aminothiazol-4-yl)-2-methoxyiminoacetamido]-3-(1-methyl-1-pyrrolidinium)methyl-3-cephem-4-carboxylat

719 mg (3.58 mmol) (Z)-2-(2-Aminothiazol-4-yl)-2-methoxyiminoessigsäure werden unter Stickstoff bei Raumtemperatur in 4.5 ml absol. Dimethylformamid gelöst. Nach Zugabe von 230 µl N-Ethyldiisopropylamin, 250 µl Tripropylamin und 310 µl Tributylamin wird auf −50°C gekühlt. 290 µl Mesylchlorid werden zugegeben und die Lösung wird 30 Minuten bei −50°C gerührt. Anschließend wird diese Lösung schnell zu einer auf 0°C gekühlten Lösung von 900 mg (2.6 mmol) 7-Amino-3-(1-methyl-1-pyrroldinium)methyl-3-cephem-4-carboxylat (× HCl) in 1.4 ml Wasser und 1.4 ml Triethylamino gegeben. Nach 5 Minuten wird die Reaktionslösung auf 400 ml Aceton gegossen. Der Niederschlag wird abgesaugt, getrocknet und über Adsorberharz HP 20 chromatographiert (Laufmittel: Acetonitrile/Wasser 5/95).

Ausbeute: 909 mg (63%).

$^1$H-NMR (DMSO-$d_6$) δ (ppm) = 9.61 (1H, d, J=9Hz, NH); 7.27 (2H, s, $NH_2$); 6.74 (1H, s, Aminothiazol); 5.65 (1H, dd, J=9Hz, J=5Hz, H-7); 5.13 (1H, d, J=5Hz, H-6); 5.02 (1H, d, J=13Hz, $CH_2$-Pyrrol.); 3.91 (1H, d, J=13Hz, $CH_2$-Pyrrol.); 3.84 (3H, s, $OCH_3$); 3.82 (1H, d, J=18Hz, S-$CH_2$); 3.44 (4H, m, Pyrrol.); 3.32 (1H, d, J=18Hz, S-$CH_2$); 2.92

(3H, s, $CH_3$ ±N—);

2.08 (4H, m, Pyrrol.).

Beispiel 3

7β-[(Z)-2-(2-Aminothiazol-4-yl)-2-methoxyiminoacetamido]-3-(trimethylammonium)methyl-3-cephem-4-carboxylat

Die Herstellung erfolgt analog Beispiel 2 aus (Z)-2-(2-Aminothiazol-4-yl)-2-methoxyiminoessigsäure und 7-Amino-3-(trimethylammonium)-methyl-3-cephem-4-carboxylat.

$^1$H-NMR (DMSO-$d_6$) δ (ppm) = 9.62 (1H, d, J=9Hz, NH); 7.27 (2H, bs, $NH_2$); 6.75 (1H, s, Thiazol); 6.66 (1H, dd, J=9Hz, J=5Hz, H-7-Lactam); 5.16 (1H, d, J=5Hz, H-6-Lactam); 5.01 (1H, d, J=14Hz, $CH_2$-Ammon.); 3.91 (1H, d, J=14Hz, $CH_2$-Ammon.); 3.84 (3H, s, $OCH_3$); 3.83 (1H, d, J=18Hz, S-$CH_2$); 3.29 (1H, d, J=18Hz, S-$CH_2$); 3.00

(9H, s, ±N—).

Beispiel 4

7β-[(Z)-2-(2-Aminothiazol-4-yl)-2-methoxyiminoacetamido]-3-(4-cyclopropylpyridinium)methyl-3-cephem-4-carboxylat

Die Herstellung erfolgt analog Beispiel 2 aus (Z)-2-(2-Aminothiazol-4-yl)-2-methoxyiminoessigsäure und 7-Amino-3-(4-cyclopropyl-pyridinium)methyl-3-cephem-4-carboxylat.

$^1$H-NMR (DMSO-$d_6$) δ (ppm) = 9.51 (1H, d, J=9Hz, NH); 9.22 (2H, d, H=6Hz, H-2,6-Py); 8.81 (2H, d, J=6Hz, H-3,5-Py); 7.20 (2H, bs, $NH_2$); 6.68 (1H, s, Thiazol); 5.62 (1H, dd, J=9Hz, J=5Hz, H-7-Lactam); 5.53 (1H, d, J=13Hz, $CH_2$-Py); 5.03 (1H, d, J=5Hz, H-6-Lactam); 4.93 (1H, d, J=13Hz, $CH_2$-Py); 3.78 (3H, s, $OCH_3$); 3.49 (1H, d, J=18Hz, S-$CH_2$); 2.98 (1H, m, Cycloprop.); 1.37 (2H, m, Cycloprop.); 1.11 (2H, m, Cycloprop.).

Beispiel 5

7β-[(Z)-2-(2-Aminothiazol-4-yl)-2-methoxyiminoacetamido]-3-isochinolinium-3-cephem-4-carboxylat

Die Herstellung erfolgt analog Beispiel 2 aus (Z)-2-(2-Aminothiazol-4-yl)-2-methoxyiminoessigsäure und 7-Amino-3-isochinolinium-3-cephem-4-carboxylat.

$^1$H-NMR (DMSO-$d_6$) δ (ppm) = 10.24 (1H, s, H-1-Isochin.); 9.51 (1H, d, J=9Hz, NH); 9.42 (1H, d, J=8Hz, H-3-Isochin.); 8.59 (1H, d, J=7Hz, Isochin.); 8.48 (1H, d, J=8Hz, Isochin.); 8.30 (1H, m, Isochin.); 8.25 (1H, m, Isochin.); 8.06 (1H, m, Isochin.); 7.19 (2H, bs, $NH_2$); 6.68 (1H, s, Thiazol); 5.72 (1H, d, J=14Hz, $CH_2$-Isochin.); 5.65 (1H, dd, J=9Hz, J=5Hz, H-7-Lactam); 5.21 (1H, d, J=14Hz, $CH_2$-Isochin.); 5.06 (1H, d, J=5Hz, H-6-Lactam); 3.76 (3H, s, $OCH_3$); 3.53 (1H, d, J=18Hz, S-$CH_2$); 3.16 (1H, d, J=18Hz, S-$CH_2$).

Beispiel 6

7β-[(Z)-2-(2-Aminothiazol-4-yl)-2-methoxyiminoacetamido]-3-(2,3-cyclopentenopyridinium)methyl-3-cephem-4-carboxylat

Die Herstellung erfolgt analog Beispiel 2 aus (Z)-2-(2-Aminothiazol-4-yl)-2-methoxyiminoessigsäure und 7-Amino-3-(2,3-cyclopentenopyridinium)methyl-3-cephem-4-carboxylat.

$^1$H-NMR (DMSO-$d_6$) δ (ppm) = 9.57 (1H, d, J=8Hz, H-6-Py); 9.29 (1H, d, J=7Hz, NH); 8.37 (1H, d, J=8Hz, H-4-Py); 7.91 (1H, m, H-5-Py); 7.24 (2H, bs, $NH_2$); 6.71 (1H, s, Thiazol); 5.65 (1H, dd, J=7Hz, J=5Hz, H-7-Lactam); 5.47 (1H, d, J=14Hz, $CH_2$-Py); 5.22 (1H, d, J=14Hz, $CH_2$-Py); 5.05 (1H, d, J=5Hz, H-6-Lactam); 3.80 (3H, s, $OCH_3$); 3.40 (1H, d, J=18Hz, S-$CH_2$); 3.39 (2H, m, Py-$CH_2$); 3.12 (3H, m, Py-$CH_2$, S-$CH_2$); 2.21 (2H, m, $—CH_2—$).

Beispiel 7

7β-[(Z)-2-(2-Aminothiazol-4-yl)-2-methoxyiminoacetamido]-3-(5,6,7,8-tetrahydrochinolinium)methyl-3-cephem-4-carboxylate

Die Herstellung erfolgt analog Beispiel 2 aus (Z)-2-(2-Aminothiazol-4-yl)-2-methoxyiminoessigsäure und 7-Amino-3-(5,6,7,8-tetrahydrochinolinium)methyl-3-cephem-4-carboxylat.

$^1$H-NMR (DMSO-$d_6$) δ (ppm) = 9.63 (1H, d, J=9Hz, NH); 9.28 (1H, d, J=7Hz, H-6-Py); 8.32 (1H, d, J=8Hz, H-4-Py); 7.94 (1H, m, H-5-Py); 7.31 (2H, bs, $NH_2$); 6.75 (1H, s, Thiazol); 5.69 (1H, dd, J=9Hz, J=5Hz, H-7-Lactam); 5.48 (1H, d, J=14Hz, $CH_2$-Py); 5.36 (1H, d, J=14Hz, $CH_2$-Py); 5.09 (1H, d, J=5Hz, H-6-Lactam); 3.83 (3H, s, $OCH_3$); 3.42 (1H, d, J=18Hz, S-$CH_2$); 3.17 (2H, m, Py-$CH_2$); 3.14 (1H, d, J=18Hz, S-$CH_2$); 2.97 (2H, m, Py-$CH_2$); 1.91 (2H, m, $—CH_2—$); 1.79 (2H, m, $—CH_2—$).

Beispiel 8

7β-[(Z)-2-(2-Aminothiazol-4-yl)-2-methoxyiminoacetamido]-3-(3-formamidopyridinium)methyl-3-cephem-4-carboxylat

Die Herstellung erfolgt analog Beispiel 2 aus (Z)-2-(2-Aminothiazol-4-yl)-2-methoxyiminoessigsäure und 7-Amino-3-(3-formamidopyridinium)methyl-3-cephem-4-carboxylat.

$^1$H-NMR (DMSO-$d_6$) δ (ppm) = 9.67 (1H, s, H-2-Py); 9.58 (1H, d, J=9Hz, NH); 9.24 (1H, d, J=7Hz, H-6-Py); 8.74 (1H, d, J=8Hz, H-4-py); 8.54 (1H, s, CHO); 8.13 (1H, m, H-5-Py); 7.24 (2H, bs, $NH_2$); 6.72 (1H, s, Thiazol); 5.73 (1H, d, J=14Hz, $CH_2$-Py); 5.68 (1H, dd, J=9Hz, J=5Hz, H-7-Lactam); 5.24 (1H, d, J=14Hz, $CH_2$-Py); 5.09 (1H, d, J=5Hz, H-6-Lactam); 3.80 (3H, s, $OCH_3$); 3.55 (1H, d, J=18Hz, S-$CH_2$); 3.15 (1H, d, J=18Hz, S-$CH_2$).

Beispiel 9

7β-[(Z)-2-(2-Aminothiazol-4-yl)-2-methoxyiminoacetamido]-3-(3-aminopyridinium)methyl-3-cephem-4-carboxylat

520 mg (1 mmol) 7β-[(Z)-2-(2-Aminothiazol-4-yl)-2-methoxyiminoacetamido]-3-(3-formamidopyridinium)methyl-3-cephem-4-carboxylat werden in 6 ml Methanol suspendiert und durch Zugabe von 0.4 ml konz. Salzsäure in Lösung gebracht. Nach 5 h wird das Methanol im Vakuum abgezogen und der Rückstand in 20 ml Wasser aufgenommen. Mit Ionenaustauscher MP 62 wird neutralisiert und dann gefriergetrocknet.

Ausbeute: 350 mg (71%).

$^1$H-NMR (DMSO-$d_6$) δ (ppm) = 9.51 (1H, d, J=9Hz, NH); 8.52 (1H, s, H-2-Py); 8.44 (1H, d, J=7Hz, H-6-Py); 7.71 (1H, dd, J=7Hz, J=8Hz, H-5-Py); 7.63 (1H, d, J=8Hz, H-4-Py); 7.26 (2H, bs, $NH_2$); 6.83 (2H, bs, $NH_2$); 6.72 (1H, s, Thiazol); 5.62 (1H, dd, J=9Hz, J=5Hz, H-7-Lactam); 5.60 (1H, d, J=13Hz, $CH_2$-Py); 5.09 (1H, d, J=5Hz); 5.08 (1H, d, J=13Hz, $CH_2$-Py); 3.81 (3H, s, $OCH_3$); 3.53 (1H, d, J=18Hz, S-$CH_2$); 3.07 (1H, d, J=18Hz, S-$CH_2$).

**Patentansprüche**

1. Verfahren zur Herstellung von Cephalosporinen der allgemeinen Formel (I)

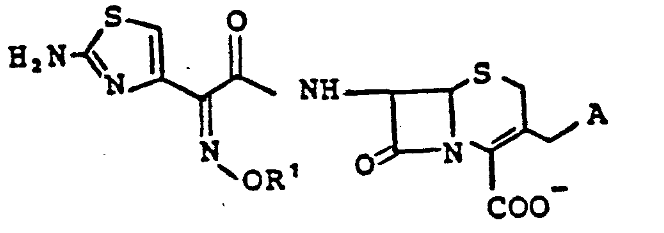

(I)

in der
$R^1$ einen $C_1$—$C_6$-Alkyl-Rest darstellt, der geradkettig, verzweigt, cyclisch und auch ungesättigt sein kann
und
A einen Rest der Formeln

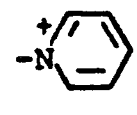
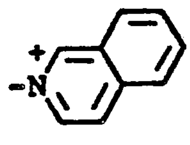
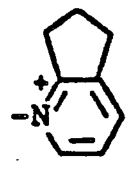
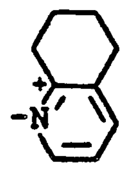

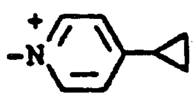
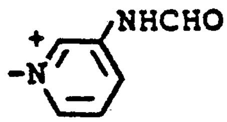
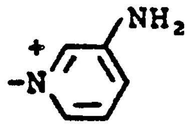

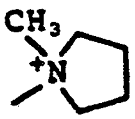
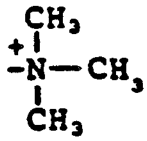

darstellt,
dadurch gekennzeichnet, daß man eine Säure der allgemeinen Formel II

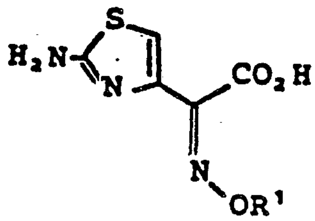
(II)

mit Methansulfonsäurechlorid in ein Anhydrid III

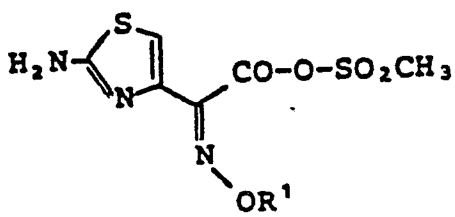
(III)

überführt und dieses mit einem 7-Aminocephalosporonat der allgemeinen Formel IV

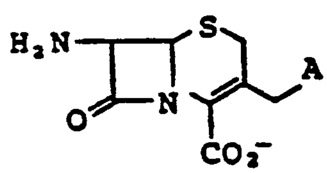
(IV)

umsetzt, wobei $R^1$ und A in Formel II, III und IV die oben angegebene Bedeutung haben.

2. Verfahren nach Anspruch 1, dadurch gekennzeichnet, daß man das Anhydrid der Formel III herstellt durch Lösen von 1 bis 1,1 Äquivalent eines Amins und der Carbonsäure der Formel II in einem Lösungsmittel und Umsetzung mit 1 bis 1,2 Äquivalenten Methansulfonsäurechlorid.

3. Verfahren nach Anspruch 2, dadurch gekennzeichnet, daß man die Umsetzung in Diethylether, Tetrahydrofuran, Acetonitrile, Aceton, Methylenchlorid, Chloroform, Dimethylformamid oder ein Gemisch dieser Lösungsmittel durchführt.

4. Verfahren nach Anspruch 2, dadurch gekennzeichnet, daß man als Amin Triethylamin, Tripropylamin, Tributylamin, Ethyldiisopropylamin, Diisopropylamin oder Gemische dieser Amine einsetzt.

5. Verfahren nach Anspruch 2, dadurch gekennzeichnet, daß man die Umsetzung bei Temperaturen zwischen −80°C und Raumtemperatur durchführt.

6. Verfahren nach Anspruch 1, dadurch gekennzeichnet, daß man die Umsetzung des Anhydrids der Formel III mit einem 7-Aminocephalosporanat der Formel IV in Dimethylformamid, Dmethylsulfoxid, Diglyme oder Wasser in Gegenwart einer Base durchführt.

7. Verfahren nach Anspruch 6, dadurch gekennzeichnet, daß man die Reaktion in Gegenwart von Triethylamin, NaOH, KOH, $NaHCO_3$ oder $K_2CO_3$ durchführt.

8. Verfahren nach Anspruch 6, dadurch gekennzeichnet, daß man die Reaktion bei −50°C bis +50°C durchführt.

9. Verfahren nach Anspruch 6, dadurch gekennzeichnet, daß man die Reaktion bei pH Werten ≥9 und Temperaturen ≤0°C durchführt.

**Revendications**

1. Procédé pour la préparation de céphalosporines de formule générale (I)

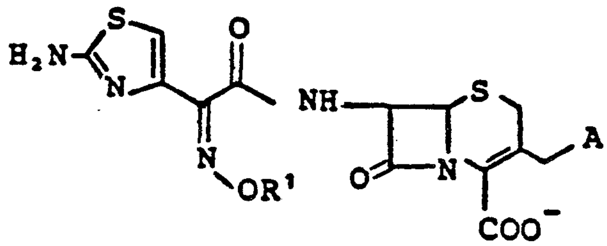

(I)

dans laquelle

$R^1$ représente un radical alkyle en $C_1$—$C_6$, qui peut être à chaîne droite, à chaîne ramifiée, cyclique et également insaturé

tandis que

A représente un radical répondant aux formules

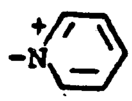
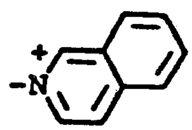
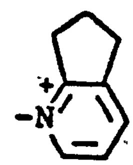
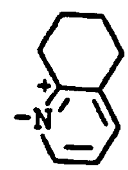
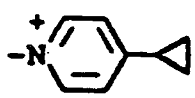
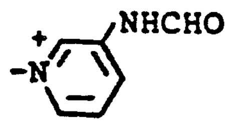
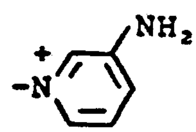
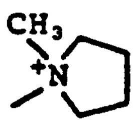
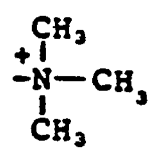

caractérisé en ce que l'on transorme un acide de formule générale II

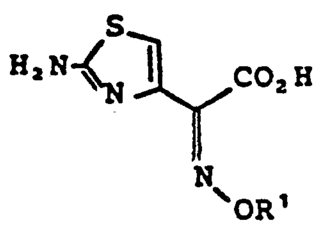

(II)

avec du chlorure de l'acide méthane-sulfonique, en un anhydride III

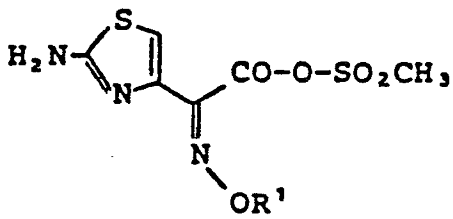

(III)

tandis que l'on fait réagir celui-ci avec un 7-aminocéphalosporanate de formule générale IV

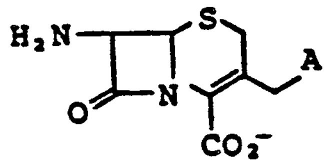

(IV)

$R^1$ et A ayant, dans les formules II, III et IV, la signification mentionnée ci-dessus.

2. Procédé selon la revendication 1, caractérisé en ce que l'on prépare l'anhydride de formule III via dissolution de 1 à 1,1 équivalent d'une amine et de l'acide carboxylique de formule II, dans un solvant, et en faisant réagir cette solution avec 1 à 1,2 équivalents de chlorure de l'acide méthane-sulfonique.

3. Procédé selon la revendication 2, caractérisé en ce que l'on effectue la transformation dans de l'éther diéthylique, du tétrahydrofuranne, de l'acétonitrile, de l'acétone, du chlorure de méthylène, du chloroforme, du diméthylformamide ou dans un mélange de ces solvants.

4. Procédé selon la revendication 2, caractérisé en ce que, comme amine, on utilise la triéthylamine, la tripropylamine, la tributylamine, l'éthyldiisopropylamine, la diisopropylamine ou des mélanges de ces amines.

5. Procédé selon la revendication 2, caractérisé en ce que l'on effectue la réaction à des températures comprises entre −80°C et la température ambiante.

6. Procédé selon la revendication 1, caractérisé en ce que l'on effectue la transformation de l'anhydride de formule III, avec un 7-aminocéphalosporanate de formule IV, dans du diméthylformamide, du diméthylsulfoxyde, du diglyme ou de l'eau, en présence d'une base.

7. Procédé selon la revendication 6, caractérisé en ce que l'on effectue la réaction en présence de triéthylamine, de NaOH, de KOH, de $NaHCO_3$ ou de $K_2CO_3$.

8. Procédé selon la revendication 6, caractérisé en ce que l'on effectue la réaction à une température allant de −50°C à +50°C.

9. Procédé selon la revendication 6, caractérisé en ce que l'on effectue la réaction à des valeurs de pH ≥9 et à des températures ≤0°C.

## Claims

1. Process for the preparation of cephalosporins of the general formula (I)

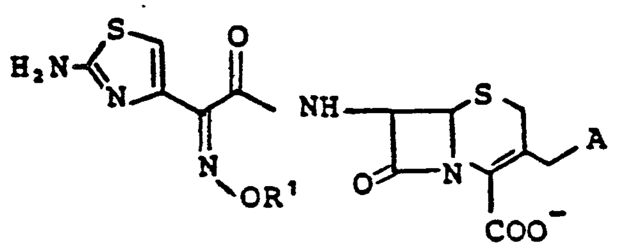

(I)

in which

$R^1$ represents $C_1$—$C_6$-alkyl radical which can be straight-chain or branched, cyclic as well as unsaturated, and

A represent a radical of the formulae

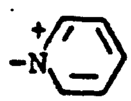
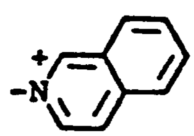
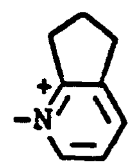
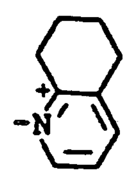

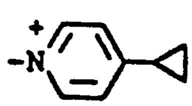
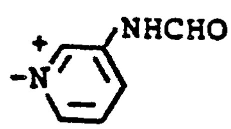
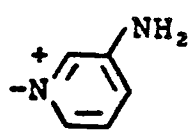

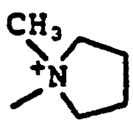
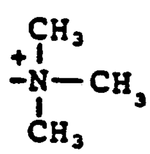

characterized in that an acid of the general formula II

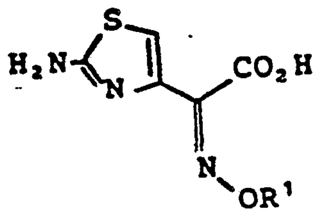

(II)

is converted, with methanesulphonyl chloride, into an anhydride III

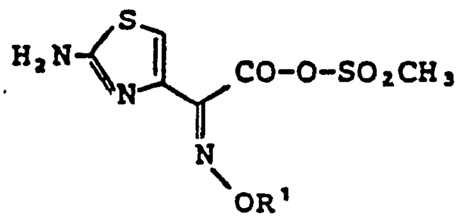

(III)

and the latter is reacted with a 7-aminocephalosporanate of the general formula IV

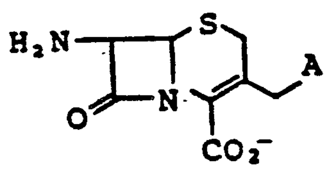

(IV)

where

$R^1$ and A in formula II, III and IV have the abovementioned meaning.

2. Process according to Claim 1, characterized in that the anhydride of the formula III is prepared by dissolving 1 to 1.1 equivalent of an amine and of the carboxylic acid of the formula II in a solvent and reacting with 1 to 1.2 equivalents of methanesulphonyl chloride.

3. Process according to Claim 2, characterized in that the reaction is carried out in diethyl ether, tetrahydrofuran, acetonitrile, acetone, methylene chloride, chloroform, dimethylformamide or a mixture of these solvents.

4. Process according to Claim 2, characterized in that the amine used is triethylamine, tripropylamine, tributylamine, ethyldiisopropylamine, diisopropylamine or mixtures of these amines.

5. Process according to Claim 2, characterized in that the reaction is carried out at temperatures between −80°C and room temperature.

6. Process according to Claim 1, characterized in that the reaction of the anhydride of the formula III with a 7-aminocephalosporanate of the formula IV is carried out in dimethylformamide, dimethyl sulphoxide, diglyme or water in the presence of a base.

7. Process according to Claim 6, characterized in that the reaction is carried out in the presence of triethylene, NaOH, KOH, $NaHCO_3$ or $K_2CO_3$.

8. Process according to Claim 6, characterized in that the reaction is carried out at −50° to +50°C.

9. Process according to Claim 6, characterized in that the reaction is carried out at pH values ≥9 and temperatures ≤0°C.